# EUROPEAN PATENT SPECIFICATION

(11) **EP 4 400 065 B1**
(45) Date of publication and mention of the grant of the patent: **18.03.2026**
(21) Application number: 23219712.9
(22) Date of filing: 22.12.2023
(51) Int. Cl.: A61B 18/14, A61B 18/00

(54) **SKIN TREATMENT APPARATUS USING RF ENERGY HAVING OVERLAP TREATMENT PREVENTION FUNCTION**
HAUTBEHANDLUNGSVORRICHTUNG MIT HF-ENERGIE MIT ÜBERLAPPUNGSBEHANDLUNGSVERHINDERUNGSFUNKTION
APPAREIL DE TRAITEMENT DE LA PEAU UTILISANT UNE ÉNERGIE RF AYANT UNE FONCTION DE PRÉVENTION DE TRAITEMENT DE CHEVAUCHEMENT

(30) Priority: 12.01.2023 KR 20230004911
(43) Date of publication of application: 17.07.2024
(73) Proprietor: Lutronic Corporation, Goyang-si, Gyeonggi-do 10534 (KR)
(72) Inventor: KO, Kwangchon, 10394 Goyang-si, Gyeonggi-do (KR)
(74) Representative: Algemeen Octrooi- en Merkenbureau B.V.

(56) References cited:
- WO-A1-2013/036761
- US-A1- 2010 249 772
- US-A1- 2014 018 783
- US-A1- 2022 314 015

## Description

### TECHNICAL FIELD

The present disclosure relates to a skin treatment device using RF energy which can prevent overlap treatment of treated tissue, a control method thereof, and a skin treatment method using the same.

### BACKGROUND

Devices for delivering RF energy to tissue for therapeutic purposes have been developed in various manners. In particular, devices that use RF energy to cause appropriate degeneration of the skin and exert a skin treatment effect through tissue regeneration have been developed.

Existing devices for skin treatment mainly use a method of delivering RF energy to a small area compared to the entire treatment area of the face. Korea Patent No. 0706155 is disclosed in relation to such a conventional treatment device using RF energy. In such existing devices, a user repeats an operation of changing the position of a handpiece in order to vary the position where the handpiece contacts the skin. However, with conventional skin treatment devices, it is difficult for a user to accurately recognize an area that had already been treated and position the handpiece on an untreated area. Additionally, if the user's skill level is low, there is a problem in that RF energy is transmitted again to tissue that has already been treated, causing overlap treatment and resulting in excessive damage to the tissue.

### [Cited Reference]

### [Patent Document]

Korea Patent No. 0706155.
US 2010/249772 teaches a device according to the preamble of present claim 1.

### SUMMARY

While the present invention is defined in claim 1, the present disclosure provides a skin treatment device using RF energy having a overlap treatment prevention function, which that can prevent overlap treatment that is a problem of conventional skin treatment devices using RF energy, a control method thereof, and a skin treatment method using the same.

Methods do not constitute a portion of the present invention, even if partially referred to as "embodiments" hereinafter.

In an aspect, a skin treatment device using RF energy having a overlap treatment prevention function includes a main body, a handpiece configured to receive RF energy from the main body, an electrode provided on the handpiece and configured to transmit the RF energy to tissue, a plurality of temperature sensors provided in the handpiece and configured to measure temperature of the target tissue, and a controller configured to determine whether at least a part of the target tissue overlaps with treated tissue on the basis of values measured by the plurality of temperature sensors when the RF energy is transmitted to the electrode.

The controller may determine that treatment for the target tissue is overlap when one or more of the values measured by the plurality of temperature sensors increase faster than other measured values.

The controller may perform control such that transmission of the RF energy is blocked upon determining that treatment for the target tissue is overlap.

The controller may perform control such that the RF energy is blocked upon determining that the target tissue overlaps with the treated tissue while the RF energy is transmitted to the electrode.

The skin treatment device may further include a substrate provided at a distal end of the handpiece, the electrode may be provided on one side of the substrate facing outward, and the plurality of sensors may be provided on the opposite side of the one side of the substrate.

At least some of the plurality of sensors may be provided at positions corresponding to outer corners of the electrode on the opposite side of the substrate.

The skin treatment device may further include a display configured to receive a signal from the controller and to display information related to a position where the target tissue overlaps with the treated tissue.

In addition, a method of controlling a skin treatment device using RF energy having a overlap treatment prevention function includes an RF energy transmission step of transmitting RF energy from an RF generator to an electrode in contact with tissue, a temperature measurement step in which a handpiece measures temperatures at a plurality of points in the tissue to which the RF energy is transmitted, and a overlap treatment determination step in which an arithmetic operation unit determines whether different change trends appear among the temperatures measured at the plurality of points.

The overlap treatment determination step may include determining that different change trends appear when a temperature of at least one point among temperature values measured at the plurality of points is determined to increase faster than temperatures of the other points.

Further, the method may further include an RF energy blocking step of blocking transmission of RF energy when it is determined that different change trends appear in the overlap treatment determination step.

Further, the overlap treatment determination step and the RF energy blocking step may be performed during execution of the RF energy transmission step.

The temperature measurement step may include receiving temperature measurement values from a plurality of sensors provided along an edge of an electrode arrangement area for transmitting the RF energy to the tissue.

The overlap treatment determination step may further include an overlap area determination step of determining a overlap treatment point in the electrode arrangement area among the plurality of points.

The method may further include an overlap area display step of displaying the overlap treatment point determined in the overlap area determination step on a display of the handpiece.

In addition, a skin treatment method using RF energy to prevent overlap treatment includes a first treatment step of treating tissue by transmitting RF energy to a first treatment area of the skin, a second treatment step of treating tissue by transmitting RF energy to a second treatment area adjacent to the first treatment area, and an overlap determination step of determining whether at least a part of the second treatment area overlaps with the first treatment area during execution of the second treatment step.

Further, the second treatment step may be stopped when it is determined that treatment is overlap in the at least a part of the second treatment area in the overlap determination step.

Further, the overlap determination step of determining whether at least a part of the second treatment area overlaps with the first treatment area may include determining that treatment is overlap when a sudden temperature increase is detected from at least one point in the second treatment area.

Further, the overlap determination step may include determining that treatment is overlap when a temperature of at least one point increases faster than temperatures of other points on the basis of temperatures measured at a plurality of points in the second treatment area.

Further, the overlap determination step may include determining overlapping on the basis of temperatures measured at a plurality of points along the edge of the second treatment area.

Further, the overlap determination step may further include an overlap area determination step of determining a direction in which treatment is overlap in the second treatment area, and an overlap area display step of displaying the direction in a treatment device for transmitting RF energy.

The skin treatment device using RF energy having a overlap treatment prevention function, the control method thereof, and the skin treatment method using the same according to the present disclosure can prevent overlap treatment during skin treatment, thereby preventing excessive tissue damage and maximizing the treatment effect.

### BRIEF DESCRIPTION OF THE DRAWINGS

FIG. 1 is a block diagram of a skin treatment device using RF energy having a overlap treatment prevention function according to a first embodiment of the present disclosure.
FIG. 2 is a graph showing temperature increase in a overlap treatment area.
FIG. 3 is a perspective view of the first embodiment.
FIG. 4 is a bottom view of a handpiece in the first embodiment.
FIG. 5 is a plan view showing a substrate in the first embodiment.
FIG. 6 is a cross-sectional view taken along I-I' in FIG. 4.
FIG. 7 is a conceptual diagram illustrating the concept of overlap treatment in the first embodiment.
FIG. 8 is a conceptual diagram illustrating the concept of preventing overlap treatment in the first embodiment.
FIG. 9 is a diagram showing a use state in the first embodiment.
FIG. 10 is a flowchart of a method of controlling the skin treatment device using RF energy having a overlap treatment prevention function according to a second embodiment of the present disclosure.
FIG. 11 is a flowchart of a control method of the skin treatment device using RF energy having a overlap treatment prevention function according to a third embodiment of the present disclosure.
FIG. 12 is a flowchart of a skin treatment method using RF energy to prevent overlap treatment according to a fourth embodiment of the present disclosure.
FIG. 13 is a flowchart of a skin treatment method using RF energy to prevent overlap treatment according to a fifth embodiment of the present disclosure.

### DETAILED DESCRIPTION

Hereinafter, a skin treatment device using RF energy having a overlap treatment prevention function, a control method thereof, and a skin treatment method using the same according to embodiments of the present disclosure will be described in detail with reference to the attached drawings. In description of the following embodiments, the name of each component may be referred to as different names in the art. Additionally, a symbol is attached to each component for convenience of description. However, content shown in the drawings in which such symbols are written does not limit each component to the scope within the drawings. Further, if a component is recognized as a component that should be included in light of the general level of technicians in the relevant technical field, the description thereof will be omitted.

The present disclosure will be described on the premise that treatment means heating the skin tissue to improve wrinkles, tone and textural changes, scars and acne scarring, sagging mucosa, overall rejuvenation, hyperhidrosis, laxity, lifting, tightening, fat reduction, etc.

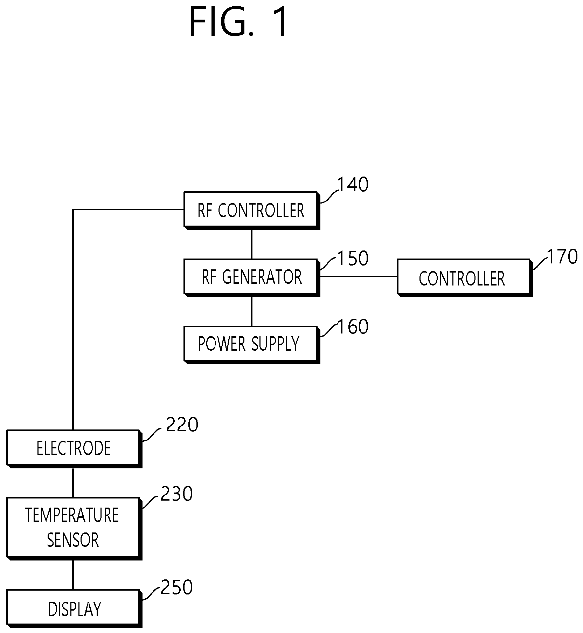

Hereinafter, a skin treatment device using RF energy having a overlap treatment prevention function according to a first embodiment of the present disclosure will be described with reference to FIGs. 1 to 9.

FIG. 1 is a block diagram of the skin treatment device using RF energy having a overlap treatment prevention function according to the first embodiment of the present disclosure.

Referring to FIG. 1, the skin treatment device using RF energy having a overlap treatment prevention function according to the first embodiment of the present disclosure includes an RF generator 150, a power supply 160, a controller 170, an electrode 220, a temperature sensor 230, and a display 250.

The RF generator 150 may be configured to receive power from the power supply 160 and generate RF energy. An RF controller 140 is configured to control at least one of the frequency, power, voltage, or electric power of RF energy generated by the RF generator 150. Meanwhile, a well-known circuit configuration may be applied to the power supply 160, the RF generator 150, and the RF controller 140.

The controller 170 may be configured to control at least one of the RF generator 150, the RF controller 140, or the power supply on the basis of user input and a value received from the temperature sensor 230 which will be described later. The controller 170 may control the RF generator 150 and the RF controller 140 such that the power and frequency of RF energy can be adjusted according to user input. In addition, the controller 170 is configured to control the RF generator 150 and the RF controller 140 based on various sensor values received when RF energy is transmitted to tissue. Additionally, the controller 170 may transmit/receive information to/from the display 250 such that various types of information can be displayed. The controller 170 may include an arithmetic operation unit and may be configured to update parameters and control RF energy according to a predetermined algorithm on the basis of a value received from the temperature sensor.

The electrode 220 may be electrically connected to the RF controller 140 and configured to receive RF energy and to transmit the RF energy to tissues in contact. A plurality of electrodes 220 may be provided and arranged in a predetermined pattern. At least a portion of the electrode 220 may be flat such that RF energy is non-invasively transmitted to tissue. The electrode 220 may function as a bipolar electrode 220 or a monopolar electrode 220.

The temperature sensor 230 is configured to measure temperature at a plurality of points while RF energy is transmitted to tissue. The temperature sensor 230 may include a plurality of temperature sensors. The plurality of temperature sensors may be configured to measure temperature at a plurality of points in a treatment area to which RF energy is transmitted. The controller 170 determines whether a different temperature change trend appears at at least one point on the basis of values measured by the plurality of temperature sensors. Here, as RF energy is transmitted, the overall temperature of the tissue increases, but when the RF energy is transmitted again to an area where tissue degeneration has already occurred due to previously transmitted RF energy, a rapid temperature increase tends to occur. Accordingly, the controller 170 controls at least one of the RF generator 150 or the RF controller 140 such that the RF energy can be blocked when a sudden temperature increase is detected in a certain area to which the RF energy is transmitted.

The display 250 may be configured to display various types of information obtained during treatment using RF energy. The display 250 may be configured to display various types of information such as user information, RF energy setting information, a current treatment mode, and the number of treatments.

Further, the controller 170 may determine whether treatment is overlap on the basis of a value measured by the temperature sensor 230. The controller 170 may be configured to transmit RF energy to tissue within a few seconds, preferably within several to hundreds of ms. The controller 170 can receive temperature measurement values from the temperature sensor during an RF energy transmission time of several to hundreds of ms, and can control RF energy by receiving temperature measurement values in real time. For example, temperature can be measured by the plurality of temperature sensors at a plurality of points for several to hundreds of ms.

Additionally, the controller 170 may be configured to measure or calculate the impedance of the tissue to control the power, voltage, current, duration, and the like of the RF energy in order to control the RF energy according to the state of the tissue.

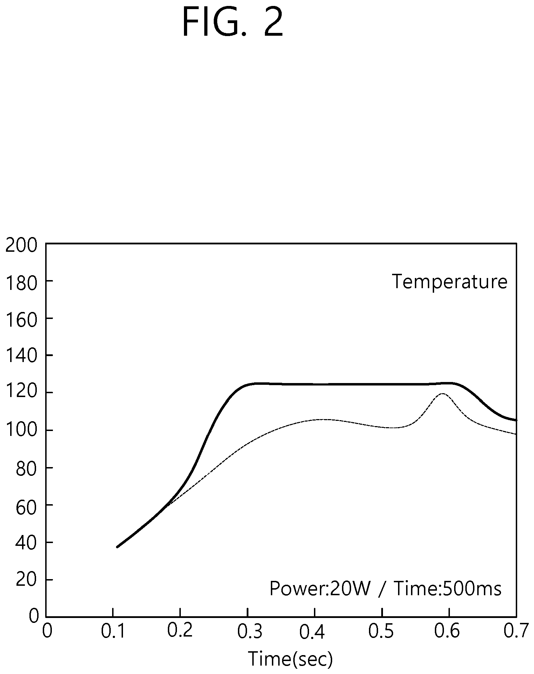

FIG. 2 is a graph showing temperature increase in a overlap treatment area.

Referring to FIG. 2, when the skin tissue may be divided into a temperature increase section, a coagulation section, and an ablation section depending on the temperature when heated. When energy is transmitted with the same power, the impedance of the tissue may vary depending on the temperature of the tissue. There is a large difference in the impedance change according to temperature of the tissue between the tissue before treatment and the tissue after treatment. Here, the tissue after treatment means tissue in a state in which at least part of the tissue has been degenerated as a result of RF energy transmission thereto. That is, when RF energy of the same power is transmitted to the tissue before and after treatment for the same time, the temperature change tendency changes as shown in FIG. 2. That is, when RF energy in the same condition is transmitted to tissue that has already been treated (denatured), the temperature of the tissue may increase more rapidly than in the tissue before treatment. This temperature increase can cause excessive tissue damage, such as ablation, and thus it is desirable to avoid overlap transmission of RF energy to the treated tissue.

The skin treatment device using RF energy having a overlap treatment prevention function according to the present disclosure is configured to determine whether the tissue to which RF energy is currently transmitted is a tissue that has already been treated through temperature measurement, and if RF energy is transmitted overlaply, to block the RF energy.

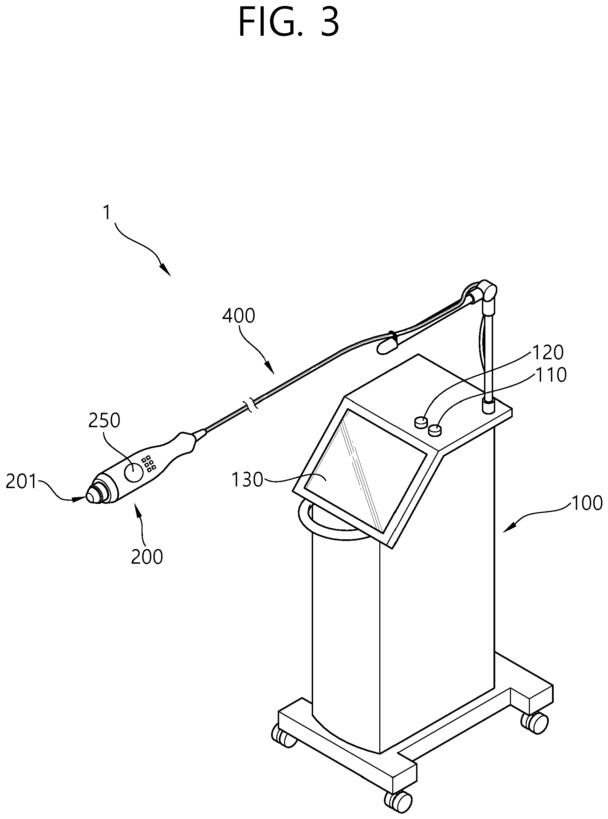

FIG. 3 is a perspective view of the first embodiment.

Referring to FIG. 3, the skin treatment device using RF energy having a overlap treatment prevention function according to an embodiment of the present disclosure includes a main body 100, a handpiece 200 held by a user to perform treatment, and a connection part 400 for connecting the main body and the piece.

The RF generator, the RF controller, and the controller (not shown) may be provided inside the main body 100. As described above, the controller generates a control input for controlling the RF generator according to a sensing value input from a sensor. At this time, the frequency of RF energy may be adjusted depending on the constitution, treatment purpose, treatment area, and the like of a patient.

A power on/off switch 110, a frequency control lever 120 for adjusting the frequency of RF energy generated from the RF generator, and a touch screen 130 for displaying various types of information including details of operation of the treatment device, receiving commands from a user, and displaying treatment information may be provided on the outer surface of the main body 100.

The handpiece 200 is connected to the main body by the connection part 400. The handpiece 200 is configured such that the user can hold and use it. A tip 201 is provided at the distal end of the handpiece 200, and the user can transmit RF energy to tissue by placing the tip in close contact with the patient's skin and manipulating the handpiece. A display 250 may be provided on one side of the handpiece to display information related to treatment or operation of the device when the user holds and operates the handpiece. The handpiece 200 transmits RF energy generated from the RF generator of the main body to a plurality of electrodes provided at the end of the tip through the connection part 400. In addition, the handpiece 200 is configured to measure the temperature when RF energy is transmitted to the tissue from a plurality of temperature sensors provided on the tip and transmit the temperature to the controller. The user may place the tip of the handpiece 200 in close contact with the skin and apply an input for transmitting RF energy. After the RF energy is transmitted to the skin tissue according to the user input, the user moves the position of the handpiece and then apply an input for transmitting the RF energy to the tissue again. The user can perform treatment on a large area of tissue, such as the entire face, by repeating this process several to hundreds of times.

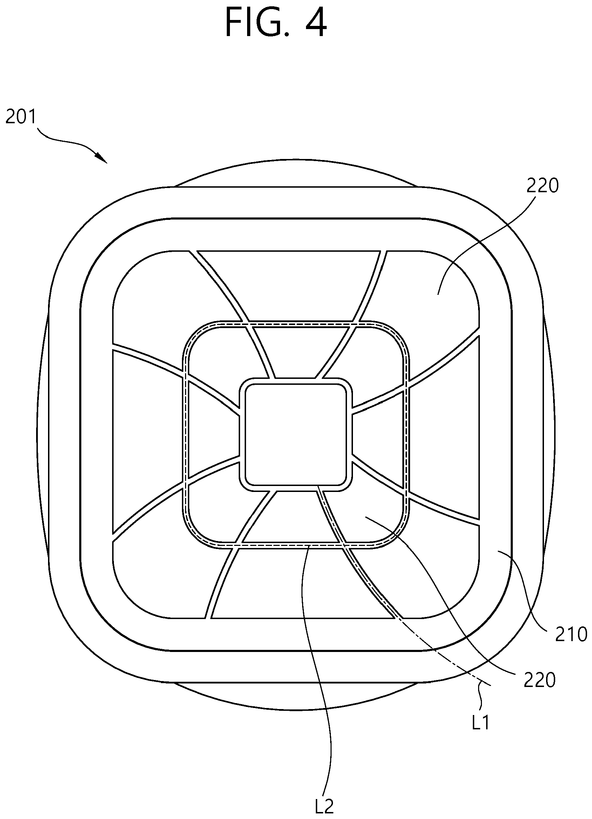

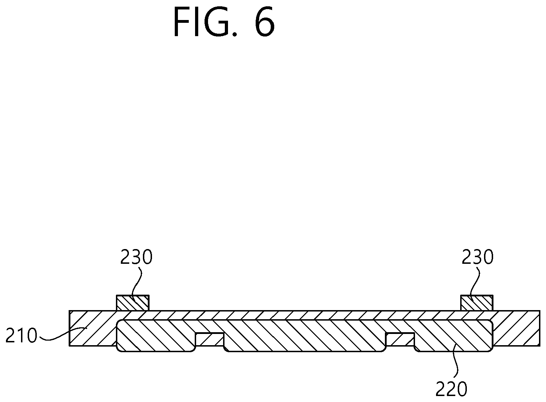

Hereinafter, electrodes and temperature sensors will be described with reference to FIGs. 4 and 6.

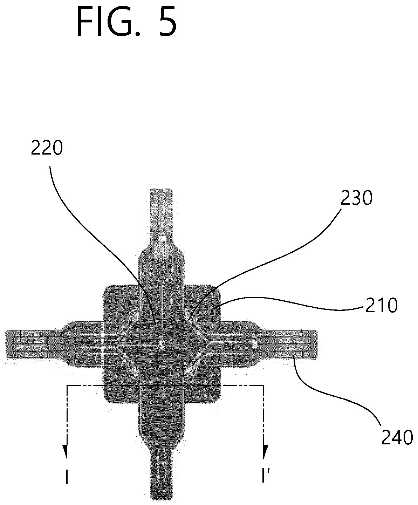

FIG. 4 is a bottom view of the handpiece in the first embodiment, FIG. 5 is a plan view showing a substrate in the first embodiment, and FIG. 6 is a cross-sectional view taken along I-I' in FIG. 4.

Referring to FIGs. 4 to 6, a plurality of electrodes 220 may be provided on the bottom of the tip 201 of the handpiece.

The plurality of electrodes 220 is configured in a planar shape and may be respectively disposed in areas divided by a virtual division line. Among the virtual division lines, first division lines are generally concentric with respect to the areas where the plurality of electrodes 220 is disposed and form a closed curve. As an example, the first division lines L1 may be formed along a rectangular path, and the corners thereof may have a predetermined curvature. Second division lines L2 are formed in a radial shape and may be curved. As an example, six second division lines may be formed in FIG. 4. The plurality of divided electrodes 220 may be configured such that RF energy is prevented from being transmitted to the edges of the electrodes 220 to cause the tissue temperature to partially excessively increase, and RF energy can be uniformly transmitted throughout the treatment area.

Referring back to FIG. 5, the inner surface equipped with the temperature sensor 230 is shown and a substrate 210 is shown in an exposed state. Here, when the substrate 210 is coupled to the handpiece, the outer surface of the substrate 210 refers to a surface that can be in contact with the skin, and the inner surface of the substrate 210 refers to a surface facing the inside of the handpiece.

The plurality of electrodes 220 may be provided on the outer surface of the substrate 210. The substrate 210 is configured in the form of a flat plate, and at least a part thereof may be flexible. The substrate 210 may be made of an insulating material to prevent RF energy transmitted to the electrodes 220 from directly affecting the temperature sensor 230. A plurality of connectors 240 is provided on the outside of the substrate 210 and can be connected to a circuit provided inside the handpiece. The plurality of connectors 240 may include a connector 240 for transmitting RF energy to each of the plurality of electrodes 220 and a connector 240 for electrically connecting to the plurality of temperature sensors 230.

The plurality of temperature sensors 230 may be provided on the inner surface of the substrate 210. The plurality of temperature sensors 230 may be provided at positions biased toward the outside of the inner surface area corresponding to the area where the plurality of electrodes 220 is disposed. As an example, four temperature sensors 230 may be provided at the corners of a square-shaped area where the electrodes 220 are disposed. Further, the temperature sensor 230 may be additionally provided at a position on the inner surface of the substrate 210 corresponding to the central portion of the area where the plurality of electrodes 220 is disposed. However, this is merely an example, and the temperature sensor 230 may be provided at a position where the temperature can be measured at the edge of the area to which RF energy is transmitted. Additionally, various numbers of temperature sensor 230 may be provided.

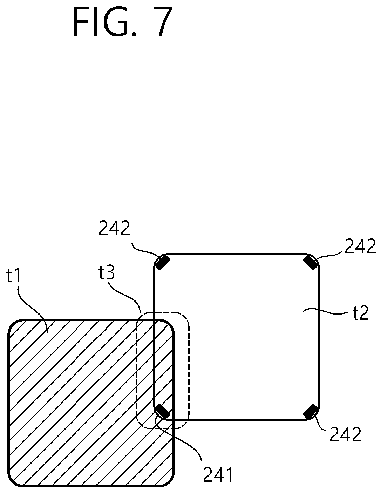

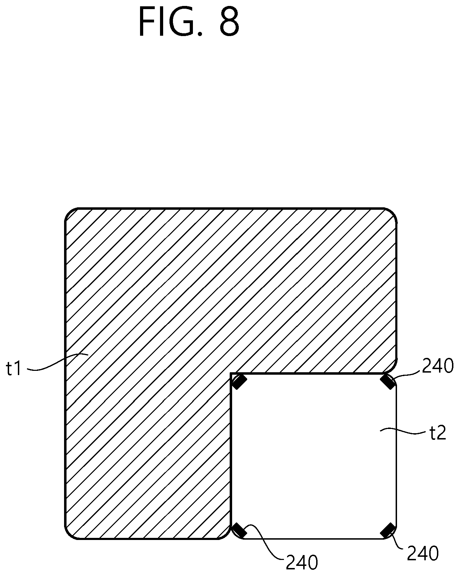

FIG. 7 is a conceptual diagram illustrating the concept of overlap treatment in the first embodiment, and FIG. 8 is a conceptual diagram illustrating the concept of preventing overlap treatment in the first embodiment.

FIG. 7 shows a first treatment area t1 and a second treatment area t2. The first treatment area is an area that has already been treated according to RF energy transmitted to the tissue. Here, the first treatment area may be an area where treatment has been completed immediately before or treatment has been performed several times ago. The second treatment area t2 is an area with which the electrodes currently come into contact and RF energy is transmitted. In this manner, when RF energy is transmitted to the second treatment area t2 in a state t3 in which a part of the second treatment area t2 overlaps with the first treatment area t1, the temperature measured by a temperature sensor 241 in 7 o'clock direction in FIG. 7 increases faster than the temperature measured by the other temperature sensors 242. The controller may ascertain that the temperature measured by one temperature sensor increases faster than the temperature measured by the remaining temperature sensors and determine that treatment is overlaply performed in a certain area on the basis of received temperature sensor values. In this case, the controller performs control such that RF energy is blocked.

Referring to FIG. 8, when a large area where treatment has been performed multiple times and thus treatment has already been completed appears, if the second treatment area t2 for the current treatment does not overlap with the first treatment area t1, treatment can be performed by transmitting preset RF energy. Here, while RF energy is transmitted to the second treatment area t2, similar temperature increase trends are detected in the temperature sensors 240. Accordingly, the controller can perform a preset treatment process and complete treatment for the second treatment area t2.

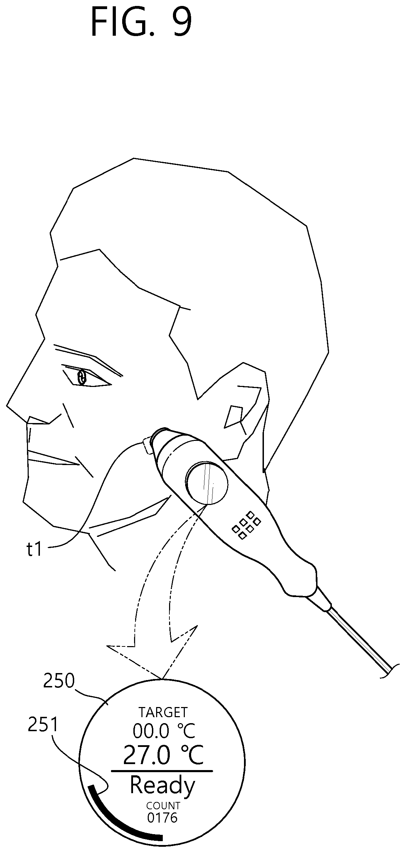

FIG. 9 is a diagram showing a use state of the first embodiment.

Referring to FIG. 9, the controller may control the temperature sensors by additionally using information on the direction in which each temperature sensor is disposed at the end of the handpiece. At this time, the position of each temperature sensor may be input to the controller in advance. The controller may determine whether at least a part of the current treatment area overlaps with the first treatment area t1 on the basis of a value measured by each temperature sensor and may also recognize a direction in which overlap treatment is being performed. Upon determining that overlap treatment is being performed, the controller may block RF energy and display (251) the direction in which overlap treatment is being performed on the display 250. Therefore, the user can check the direction in which overlap treatment is performed based on the handpiece, adjust the handpiece to a position where treatment is not overlaply performed, and then transmit RF energy to avoid overlap treatment. The controller can perform arithmetic operations and control such that operations of ascertaining overlap treatment and displaying a position at which overlap treatment is performed are overlaply carried out during RF energy shot performed several to hundreds of times.

Hereinafter, a method of controlling the skin treatment device using RF energy having a overlap treatment prevention function, which is another embodiment of the present disclosure, will be described.

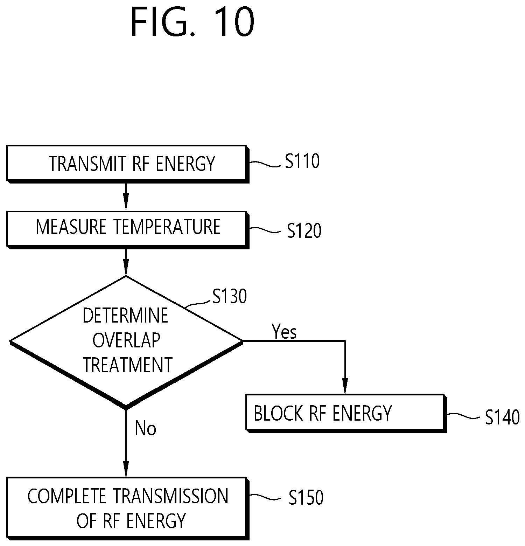

FIG. 10 is a flowchart of a method of controlling the skin treatment device using RF energy having a overlap treatment prevention function according to a second embodiment of the present disclosure.

Referring to FIG. 10, the method of controlling the skin treatment device using RF energy having a overlap treatment prevention function according to a second embodiment of the present disclosure may include an RF energy transmission step S110, a temperature measurement step S120, a overlap treatment determination step S130, and an RF energy blocking step S140.

The RF energy transmission step S110 corresponds to a step of transmitting RF energy to a plurality of electrodes in contact with the skin. The RF energy transmission step S110 may be performed based on a value input by a user. The value input by the user may be a value related to transmission of RF energy, such as the power and frequency of RF energy. The RF energy transmission step S110 may be controlled in real time. As an example, the output or application time of RF energy can be controlled on the basis of the impedance of the target tissue in contact with the electrodes.

The temperature measurement step S120 corresponds to a step of measuring temperature at a plurality of points in the tissue to which RF energy is transmitted by a plurality of temperature sensors during execution of the RF energy transmission step S110. In this step, when RF energy is transmitted to the tissue through the electrodes while the handpiece is in contact with the skin, temperature values at various points measured through the handpiece due to an increase in the temperature of the tissue are received.

The overlap treatment determination step S130 is a step of determining whether the target tissue to which RF energy is currently transmitted is tissue that has already been treated. The controller determines whether treatment is overlaply performed on the basis of received temperature measurement values. Specifically, the controller receives temperature measurement values of various points on the surface of the tissue being heated by RF energy transmitted thereto in real time, and if the temperature measurement value of at least one point increases faster than the temperature measurement values of other points, determines that overlap treatment is being performed.

The RF energy blocking step S140 corresponds to a step of immediately blocking RF energy upon determining that at least a part of the target tissue to which the RF energy is currently transmitted has already been treated. Upon determining that the current treatment area overlaps a treated area in the above-described overlap treatment determination step S130, the controller can control the RF generator and/or the RF controller, or a circuit related thereto to block the RF energy transmitted to the electrodes.

If the temperature measurement values measured at the plurality of points change similarly in real time in the overlap treatment determination step S130, the controller determines whether an increase rate exceeds a threshold value first. If all measured temperatures change in a similar trend and do not exceed the threshold value, the controller determines that the target tissue to which RF energy is currently transmitted is determined to be treated for the first time. Then, the controller complete treatment for 1 shot of RF energy. However, if the measured temperatures change in a similar trend but have a change tendency to exceed the threshold value, the controller can determine that the entire target tissue to which RF energy is currently transmitted is overlaply treated and block the RF energy.

In addition, if it is determined in the overlap treatment determination step S130 that the area to which RF energy is currently transmitted is treated for the first time, the transmission of RF energy may be completed under predetermined conditions using preset parameters (S150).

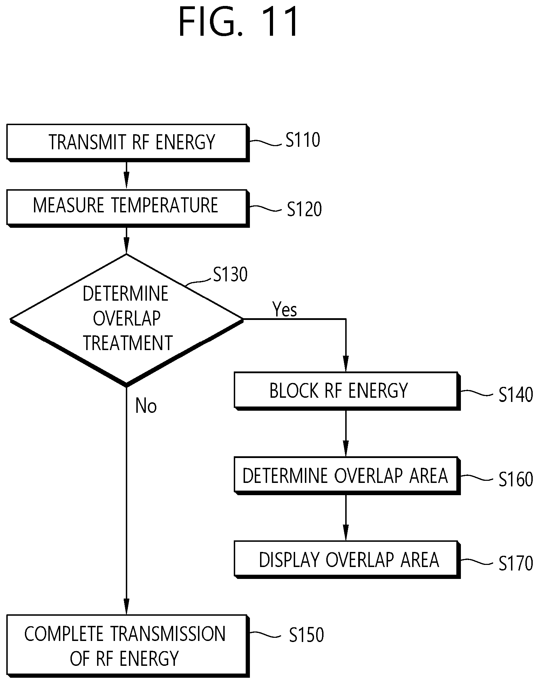

FIG. 11 is a flowchart of a method of controlling the skin treatment device using RF energy having a overlap treatment prevention function according to a third embodiment of the present disclosure.

This embodiment may also include the same steps as the second embodiment, and description of the same steps will be omitted and only different steps will be described.

Referring to FIG. 11, the method of controlling the skin treatment device using RF energy having a overlap treatment prevention function according to the third embodiment of the present disclosure may further include an overlap area determination step S160 and an overlap area display step S170.

The overlap area determination step S160 is a step in which the controller determines which part is overlaply treated. Signals received by the controller from a plurality of temperature sensors can be distinguished from each other. The positions of the temperature sensors may be stored in advance in the controller. Upon determining that the target tissue to which RF energy is currently transmitted has undergone overlap treatment, the controller determines a temperature sensor that has detected a sudden temperature increase.

The overlap area display step S170 corresponds to a step of displaying an overlap area determined by the controller on the display. The overlap area display step S170 may be performed by indicating a direction along the circumference of the handpiece. As an example, display may be performed such that the direction in which each temperature sensor is disposed based on the longitudinal central axis of the handpiece is the same as the direction in which the overlap area is displayed when the user views the display.

Meanwhile, if it is determined that treatment is overlaply performed in the overlap treatment determination step 130, the overlap area determination step S160, the overlap area display step 170, and the RF energy blocking step S140 may be performed immediately.

The methods of controlling the skin treatment device using RF energy having a overlap treatment prevention function according to the present disclosure described above with reference to FIGs. 10 and 11 may be performed using the skin treatment device using RF energy described with reference to FIGs. 1 to 9.

Hereinafter, a skin treatment method using RF energy to prevent overlap treatment according to another embodiment of the present disclosure will be described.

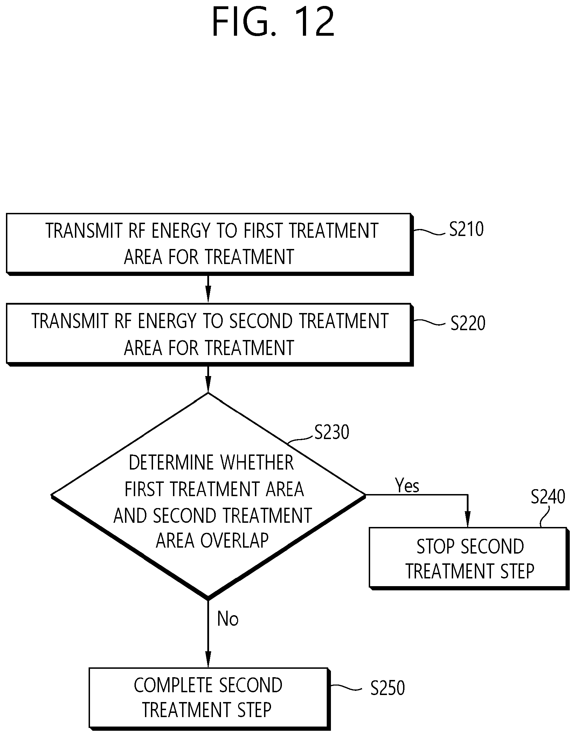

FIG. 12 is a flowchart of a skin treatment method using RF energy to prevent overlap treatment according to a fourth embodiment of the present disclosure.

Referring to FIG. 12, the skin treatment method using RF energy to prevent overlap treatment according to the fourth embodiment of the present disclosure may include a first treatment step S210 of transmitting RF energy to a first treatment area for treatment, a second treatment step S220 of transmitting RF energy to a second treatment area for treatment, a step S230 of determining whether the first treatment area and the second treatment area overlap, and a second treatment interruption step S240.

A user may treat the skin by overlaply transmitting RF energy several to hundreds of times. At this time, when RF energy is overlaply transmitted, treatment may be performed by changing areas to which the RF energy is transmitted. At this time, an area to which RF energy is transmitted at the time of performing treatment one time is smaller than the entire treatment target area. Therefore, the user can complete treatment of the entire area by transmitting RF energy each time positions at which RF electrodes are in close contact with the skin are changed.

The first treatment step S210 in which RF energy is transmitted to the first treatment area for treatment corresponds to a step of attaching electrodes to a certain area of the skin and transmitting RF energy to treat target tissue. The tissue to which RF energy has been transmitted is heated to cause tissue degeneration.

The second treatment step S220 in which RF energy is transmitted to the second treatment area for treatment corresponds to a step in which the user changes the position of the handpiece for transmitting RF energy to the second treatment area and transmits the RF energy. The second treatment area is the next treatment target after the first treatment step. The user recognizes previously treated areas through experience, ascertains changes in the skin surface, and attaches electrodes to an area where treatment has not been performed. Thereafter, RF energy is transmitted to the second treatment area according to user input.

The step S230 of determining whether the first treatment area and the second treatment area overlap corresponds to a step of measuring temperature in real time at various points in the second treatment area while the RF energy is being transmitted and determining whether treatment is overlap. This step can be performed continuously within several to hundreds of ms during one RF energy shot. In this step, it is determined whether treatment is overlap on the basis of whether a temperature change tendency is different at at least one point among several points in the second treatment area. Specifically, in the first treatment area where treatment has already been performed, tissue degeneration has occurred and thus the temperature rapidly increases even when the same RF energy is transmitted thereto. Therefore, the controller determines that the second treatment area overlaps the first treatment area if the real-time temperature measurement value of at least one point is higher than those of other points.

The second treatment interruption step S240 is performed when the controller determines that at least a part of the treatment area overlaps in the above-described step S230 of determining whether the first treatment area and the second treatment area overlaps.

A second treatment completion step S250 is a step of completing treatment for the second treatment area by applying RF energy under preset conditions upon determining that the second treatment area does not overlap with the first treatment area in the above-described step S230 of determining whether the first treatment area and the second treatment area overlap.

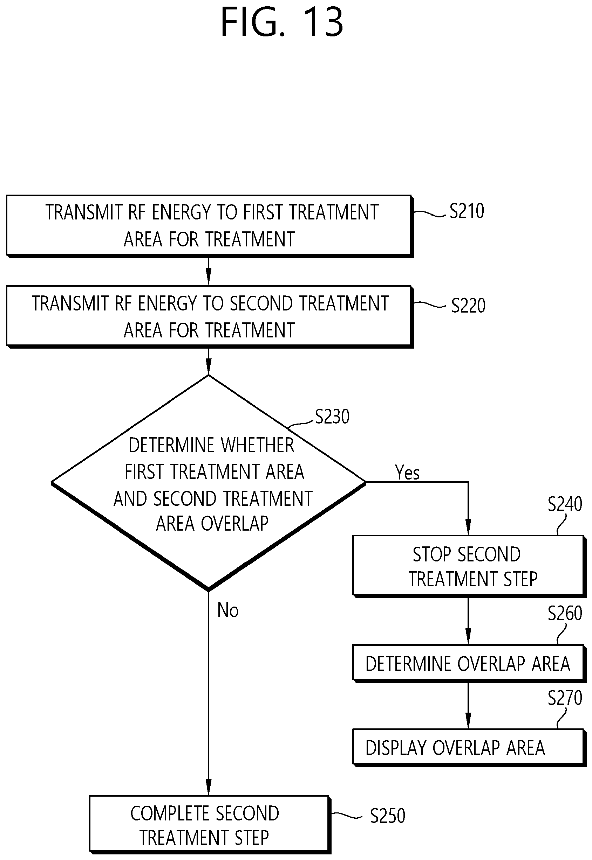

FIG. 13 is a flowchart of a skin treatment method using RF energy to prevent overlap treatment according to a fifth embodiment of the present disclosure.

FIG. 13 also includes the same steps as the fourth embodiment described above, and different steps will be described below.

Referring to FIG. 13, if it is determined that at least a part of the second treatment area overlaps with the first treatment area in the overlap determination step S230, the second treatment step is stopped (S250), and an overlap area determination step S260 and an overlap area display step S270 may be performed.

The overlap area determination step S260 is a step in which the controller determines a direction in which overlap treatment has been performed on the handpiece in the second treatment areas. This step can be performed by determining which temperature sensor among the plurality of temperature sensors provided in the handpiece has detected rapidly increased temperature.

The overlap area display step S270 corresponds to a step of displaying the area of the second treatment area overlapping with the first treatment area on the display of the handpiece or the main body of the RF treatment device. After the overlap area is determined, it is possible to display the position at which overlap treatment has been performed through the handpiece to the user and move the position of the handpiece such that the user can escape the overlap area. Thereafter, if the user starts treatment again, the second treatment step is completed (S250), or if at least a part of the second treatment area is determined to overlap with the first treatment area, the second treatment interruption step S240 to the overlap area display step S270 may be performed.

The skin treatment method using RF energy to prevent overlap treatment according to the present disclosure described above with reference to FIGs. 12 and 13 can be performed using the skin treatment device using RF energy described above with reference to FIGs. 1 to 8.

According to the skin treatment device using RF energy having a overlap treatment prevention function, the control method thereof, and the skin treatment method using the same according to the present disclosure described above, it is possible to automatically block RF energy in case of overlap treatment and make a user aware of an overlap area.

## Claims

1. A skin treatment device using RF energy having a overlap treatment prevention function, comprising:
a main body (100);
a handpiece (200) configured to receive RF energy from the main body (100);
an electrode (220) provided on the handpiece (200) and configured to transmit the RF energy to tissue;
a plurality of temperature sensors (230, 241, 242) provided in the handpiece (200) and configured to measure temperature of the target tissue; and
**characterized by** a controller (170) configured to determine whether at least a part of the target tissue overlaps with treated tissue on the basis of values measured by the plurality of temperature sensors (230, 241, 242),
wherein the device further comprises a display (250) configured to receive a signal from the controller (170) and to display information related to a position where the target tissue overlaps with the treated tissue.

2. The skin treatment device of claim 1, wherein the controller is configured to determine that treatments for the target tissue overlap when one or more of the values measured by the plurality of temperature sensors increase faster than other measured values when the RF energy is transmitted to the electrode.

3. The skin treatment device of claim 2, wherein the controller is configured to perform control such that transmission of the RF energy is blocked upon determining that treatments for the target tissue overlap.

4. The skin treatment device of claim 3, wherein the controller is configured to perform control such that the RF energy is blocked upon determining that the target tissue overlaps with the treated tissue while the RF energy is transmitted to the electrode.

5. The skin treatment device of claim 4, further comprising a substrate provided at a distal end of the handpiece,
wherein the electrode is provided on one side of the substrate facing outward, and the plurality of sensors is provided on the opposite side of the one side of the substrate.

6. The skin treatment device of claim 4, wherein at least some of the plurality of temperature sensors are provided at positions corresponding to outer corners of the electrode on the opposite side of the substrate.

## Patentansprüche

1. Hautbehandlungsvorrichtung, die HF-Energie verwendet, mit einer Überlappungsbehandlungsverhinderungsfunktion, umfassend:
einen Hauptkörper (100);
ein Handstück (200), das dazu ausgelegt ist, HF-Energie von dem Hauptkörper (100) zu empfangen;
eine Elektrode (220), die an dem Handstück (200) bereitgestellt ist und dazu ausgelegt ist, die HF-Energie an Gewebe zu übertragen;
mehrere Temperatursensoren (230, 241, 242), die in dem Handstück (200) bereitgestellt sind und dazu ausgelegt sind, die Temperatur des Zielgewebes zu messen; und
**gekennzeichnet durch** eine Steuerung (170), die dazu ausgelegt ist, basierend auf Werten, die durch die mehreren Temperatursensoren (230, 241, 242) gemessen werden, zu bestimmen, ob zumindest ein Teil des Zielgewebes mit behandeltem Gewebe überlappt,
wobei die Vorrichtung ferner eine Anzeige (250) umfasst, die dazu ausgelegt ist, ein Signal von der Steuerung (170) zu empfangen und Informationen in Bezug auf eine Position anzuzeigen, an der sich das Zielgewebe mit dem behandelten Gewebe überlappt.

2. Hautbehandlungsvorrichtung nach Anspruch 1, wobei die Steuerung dazu ausgelegt ist, zu bestimmen, dass Behandlungen für das Zielgewebe überlappen, wenn einer oder mehrere der durch die mehreren Temperatursensoren gemessenen Werte schneller als andere gemessene Werte zunehmen, wenn die HF-Energie an die Elektrode übertragen wird.

3. Hautbehandlungsvorrichtung nach Anspruch 2, wobei die Steuerung dazu ausgelegt ist, eine Steuerung derart durchzuführen, dass die Übertragung der HF-Energie blockiert wird, wenn bestimmt wird, dass sich Behandlungen für das Zielgewebe überlappen.

4. Hautbehandlungsvorrichtung nach Anspruch 3, wobei die Steuerung dazu ausgelegt ist, eine Steuerung derart durchzuführen, dass die HF-Energie blockiert wird, wenn bestimmt wird, dass das Zielgewebe mit dem behandelten Gewebe überlappt, während die HF-Energie an die Elektrode übertragen wird.

5. Hautbehandlungsvorrichtung nach Anspruch 4, ferner umfassend ein Substrat, das an einem distalen Ende des Handstücks bereitgestellt ist,
wobei die Elektrode auf einer Seite des Substrats bereitgestellt ist, die nach außen weist, und die mehreren Sensoren auf der gegenüberliegenden Seite der einen Seite des Substrats bereitgestellt sind.

6. Hautbehandlungsvorrichtung nach Anspruch 4, wobei zumindest einige der mehreren Temperatursensoren an Positionen bereitgestellt sind, die äußeren Ecken der Elektrode auf der gegenüberliegenden Seite des Substrats entsprechen.

## Revendications

1. Dispositif de traitement de la peau utilisant une énergie RF ayant une fonction de prévention de traitement de chevauchement, comprenant :
un corps principal (100) ;
une pièce à main (200) configurée pour recevoir l'énergie RF du corps principal (100) ;
une électrode (220) prévue sur la pièce à main (200) et configurée pour transmettre l'énergie RF au tissu ;
une pluralité de capteurs de température (230, 241, 242) prévus dans la pièce à main (200) et configurés pour mesurer la température du tissu cible ; et
**caractérisé par** un dispositif de commande (170) configuré pour déterminer si au moins une partie du tissu cible chevauche le tissu traité sur la base des valeurs mesurées par la pluralité de capteurs de température (230, 241, 242),
dans lequel le dispositif comprend en outre un dispositif d'affichage (250) configuré pour recevoir un signal du dispositif de commande (170) et pour afficher des informations relatives à une position où le tissu cible chevauche le tissu traité.

2. Dispositif de traitement de la peau selon la revendication 1, dans lequel le dispositif de commande est configuré pour déterminer que les traitements pour le tissu cible se chevauchent lorsqu'une ou plusieurs des valeurs mesurées par la pluralité de capteurs de température augmentent plus rapidement que les autres valeurs mesurées lorsque l'énergie RF est transmise à l'électrode.

3. Dispositif de traitement de la peau selon la revendication 2, dans lequel le dispositif de commande est configuré pour réaliser une commande de sorte que la transmission de l'énergie RF soit bloquée lors de la détermination du fait que les traitements pour le tissu cible se chevauchent.

4. Dispositif de traitement de la peau selon la revendication 3, dans lequel le dispositif de commande est configuré pour réaliser une commande de sorte que l'énergie RF soit bloquée lors de la détermination du fait que le tissu cible chevauche le tissu traité pendant que l'énergie RF est transmise à l'électrode.

5. Dispositif de traitement de la peau selon la revendication 4, comprenant en outre un substrat prévu au niveau d'une extrémité distale de la pièce à main,
dans lequel l'électrode est prévue sur un côté du substrat tourné vers l'extérieur, et la pluralité de capteurs est prévue sur le côté opposé au côté du substrat.

6. Dispositif de traitement de la peau selon la revendication 4, dans lequel au moins certains de la pluralité de capteurs de température sont prévus à des positions correspondant à des coins externes de l'électrode sur le côté opposé du substrat.
